# FASCICULE DE BREVET EUROPEEN

(11) **EP 1 023 914 B1**
(45) Date de publication et mention de la délivrance du brevet: **09.06.2004**
(21) Numéro de dépôt: 00101559.3
(22) Date de dépôt: 27.01.2000
(51) Int. Cl.: A61M 39/28

(54) **Pince pour obturer un tube souple**
Vorrichtung zum Abklemmen eines biegsamen Schlauches
Clamping device to compress a flexible tube

(30) Priorité: 28.01.1999 FR 9901132
(43) Date de publication de la demande: 02.08.2000
(73) Titulaire: Fresenius Vial SA, 38590 Brézins (FR)
(72) Inventeur: Plazy, Jean-Marc, 38240 Meylan (FR)
(74) Mandataire: Vièl, Christof

(56) Documents cités:
- EP-A- 0 150 666
- WO-A-86/07625
- WO-A-93/24173
- FR-A- 1 529 535
- US-A- 4 634 092

## Description

La présente invention concerne une pince pour obturer un tube souple, composée d'un boîtier, d'un bouton poussoir pénétrant partiellement à l'intérieur du boîtier et d'un dispositif de ressort, ledit boîtier comprenant un socle et des parois latérales et étant pourvu de deux ouvertures pratiquées dans ses parois latérales, ledit bouton poussoir étant pourvu d'un conduit transversal de sorte que lorsque ledit conduit du bouton poussoir et les ouvertures latérales du boîtier sont alignés il se forme un canal destiné à recevoir un tube souple, ledit dispositif de ressort, prenant appui sur le socle du boîtier, repoussant le bouton poussoir de façon à pincer le tube souple.

Il est courant, pour obturer des tubes souples, d'employer des pinces n'ayant que deux positions, à savoir la position ouverte et la position fermée. Ces pinces sont employées principalement en médecine pour les perfusions, mais elles trouvent également des applications dans d'autres domaines comme celui de la chimie.

L'usage de la perfusion en médecine est très répandu, qu'il s'agisse d'administrer à un patient du sang, du sérum ou une solution médicamenteuse. La solution circule de la source vers le patient à travers un tube dans lequel le débit du liquide est régulé soit par le simple effet de la gravité soit par l'usage d'une pompe placée entre la source de liquide et le patient.

Il peut être nécessaire d'interrompre le débit de liquide dans la perfusion, par exemple pour permettre au personnel médical d'installer une pompe ou de changer la source de liquide. Pour cela, on recourt à des pinces qui compriment le tube et interrompent le débit de liquide. Le tube doit être souple au moins dans la section destinée à recevoir la pince. En effet, le matériau choisi doit être apte à supporter les déformations imposées par la pince.

Ces pinces étant installées autour du tube, il est nécessaire de les positionner avant l'installation de la perfusion. En position de repos, elles obturent le tube en le pinçant, ce qui interrompt la circulation du liquide. Pour permettre au liquide de circuler à nouveau, il est nécessaire d'exercer une pression sur le bouton poussoir afin d'aligner les ouvertures du boîtier et le conduit du bouton poussoir. Il va de soi qu'il n'est pas question pour le personnel médical d'appuyer sur le bouton durant toute la perfusion. Il est donc nécessaire de trouver un moyen de maintenir alignés les ouvertures et le conduit transversal.

Le brevet US 4,634,092 propose de pratiquer des orifices dans le boîtier et le bouton poussoir de telle sorte que, lorsque les ouvertures du boîtier et le conduit du bouton poussoir sont alignés, c'est-à-dire en position ouverte, les orifices pratiqués dans le boîtier et dans le bouton sont également alignés. Une goupille est alors introduite dans les orifices ainsi alignés, maintenant la pince dans la position ouverte de façon durable.

Cette solution présente plusieurs inconvénients. Tout d'abord, deux mains sont nécessaires pour d'une part exercer la pression nécessaire à l'alignement des orifices et d'autre part faire pénétrer la goupille dans le conduit ainsi formé. Cette pièce n'étant pas fixée à la pince, elle risque de plus d'être égarée, rendant la pince pratiquement inutilisable.

La demande de brevet européen EP 0 150 666 présente un dispositif obturateur constitué principalement d'un boîtier dans lequel passe un tube qui peut être comprimé à l'aide d'un bouton poussoir. Un ressort tend à maintenir le bouton poussoir en position fermée. Afin de maintenir le dispositif obturateur en position ouverte, il est prévu autour du bouton poussoir un anneau pivotant. Cet anneau présente deux encoches latéralement opposées dans lesquelles pénètrent deux crochets fixés sur le bouton poussoir. En position normale, les deux crochets sont alignés avec les encoches de sorte que le bouton poussoir peut coulisser et sous l'action du ressort se placer en position obturée. Si par contre les deux crochets ne sont plus alignés avec les encoches de l'anneau suite au pivotement de celui-ci, les crochets restent bloqués sur l'extrémité de l'anneau de sorte que le bouton poussoir est immobilisé dans cette position ouverte. Pour refermer le dispositif obturateur, il est nécessaire de refaire pivoter l'anneau afin d'aligner les crochets et les encoches. Afin de bloquer le bouton poussoir en position ouverte, il est nécessaire tout d'abord d'agir sur le bouton poussoir pour l'amener en position ouverte puis tout en maintenant le bouton poussoir dans cette position de faire pivoter l'anneau. Cette opération a donc l'inconvénient de nécessiter deux opérations successives.

Dans les deux cas envisagés précédemment, le dispositif de ressort assurant normalement la fermeture du tube se trouve comprimé lorsque la pince est maintenue en position ouverte, ce qui diminue la durée de vie des pinces ainsi conçues.

L'invention a pour but de remédier à ces inconvénients en proposant une pince pouvant être mise en position ouverte prolongée d'une seule main, dont les différentes pièces sont libres de toute contrainte lorsque la pince est utilisée dans cette position ouverte et dont les pièces nécessaires au bon fonctionnement ne puissent pas être égarées.

Ce but est atteint selon l'invention par le fait que le socle du boîtier peut être séparé du reste du boîtier, permettant ainsi de supprimer de façon réversible la pression exercée sur le bouton poussoir par le dispositif de ressort.

Lorsque la pince doit être ouverte pour une courte durée, le personnel peut appuyer sur le bouton poussoir, provoquant ainsi l'alignement du canal dans lequel passe le tube de perfusion. Si l'ouverture doit être maintenue plus longtemps, le socle est séparé des parois du boîtier, libérant le dispositif de ressort qui n'exerce plus alors de poussée sur le bouton poussoir. Les pièces du dispositif ne sont soumises à aucune contrainte, ce qui permet d'allonger la durée de vie de telles pinces. Lorsque le moment est venu de refermer la pince, il suffit de fixer à nouveau le socle sur le boîtier.

Un autre avantage de l'invention réside dans le fait qu'il est possible de stériliser l'ensemble formé par le tube et la pince lorsque le socle est séparé du reste du boîtier. De plus, le tube ne risque pas d'être collé durant le stockage.

Afin de faciliter la séparation du socle, il est conforme à l'invention de munir le socle et le boîtier d'un dispositif de verrouillage pourvu de pattes extérieures permettant de séparer le socle du boîtier lorsqu'une pression est exercée sur lesdites pattes. Il est donc possible de mettre la pince en position d'ouverture prolongée à l'aide d'une seule main.

Pour réduire les risques d'ouverture accidentelle, il est prévu de munir le socle et le boîtier d'un dispositif de verrouillage ne pouvant être déverrouillé qu'en exécutant deux opérations consécutives indépendantes qui ne peuvent être accomplies simultanément. Les dimensions du dispositif de verrouillage, tel que celui évoqué au paragraphe précédent, peuvent être choisies de telle manière que l'appui sur les pattes, même simultané, ne libère qu'un côté du support, la pince restant occlusive. Un deuxième appui est nécessaire pour libérer l'autre côté. Ainsi, il n'est pas possible de déverrouiller les deux côtés en même temps.

Il est avantageux de placer la pince dans la cavité d'une pompe dont le mécanisme assure l'ouverture automatique de celle-ci uniquement lorsque l'ensemble formé par la pince et le tube est correctement positionné dans ladite pompe. Ces pompes, telle que celle décrite dans la demande WO 96/30679 sont munies d'un couvercle qui en position fermée appuie sur le bouton poussoir, libérant ainsi la circulation du liquide dans le tube. Cette ouverture automatique ne se produit que lorsque l'ensemble du dispositif de perfusion est correctement installé dans la pompe. Si par hasard, le tube était mal positionné dans la pompe, le couvercle ne se fermerait pas correctement et la pince se refermerait automatiquement. Cette disposition prévient le risque de « free-flow »dans lequel la pompe ne pouvant réguler correctement le débit du liquide, celui-ci s'écoule librement au risque de provoquer de graves accidents médicaux. La pince conforme à l'invention peut donc selon les besoins soit être maintenue ouverte en exerçant une pression continue sur le bouton poussoir à l'aide du boîtier de la pompe, soit être ouverte en libérant le socle du boîtier de la pince lorsque celle-ci n'est plus placée dans la pompe. Il n'est par conséquent pas nécessaire d'interrompre la perfusion lorsque le tube doit être sorti de la pompe, par exemple pour permettre le transport du malade.

Conformément à l'invention, il est préférable de munir le boîtier et le bouton poussoir d'un dispositif empêchant la rotation du bouton poussoir sur son axe de poussé à l'intérieur du boîtier. Le bouton poussoir ne pouvant pivoter à l'intérieur du boîtier, le conduit transversal du bouton ne risque pas de faire un angle par rapport à l'axe des ouvertures des parois latérales du boîtier. Cela évite de devoir tourner le bouton poussoir sur son axe jusqu'à alignement des ouvertures latérales du boîtier et du conduit du bouton poussoir au moment où la pince est montée sur le tube souple. Ce dispositif peut consister à donner une forme non circulaire à la coupe longitudinale du boîtier et du bouton poussoir, perpendiculairement à l'axe de ce dernier.

Un autre mode de réalisation de l'invention consiste à fixer le dispositif de ressort par l'une de ses extrémités au socle du boîtier et par son autre extrémité à la face interne du bouton poussoir. Ainsi, lorsque la pince est positionnée sur le tube en position d'ouverture prolongée, le socle reste liée à la pince et ne risque pas d'être égaré.

Dans un autre développement avantageux de l'invention, le dispositif de ressort est constitué de deux ressorts hélicoïdaux parallèles situés de part et d'autre du conduit transversal du bouton poussoir. Cette solution a l'avantage d'utiliser au mieux le volume intérieur de la pince. Si de plus, les ressorts prennent appui sur des ailes du bouton poussoir en retrait par rapport au conduit transversal, la profondeur totale de la pince peut être diminuée la rendant ainsi plus compacte. Une autre variante consiste à remplacer les deux ressorts hélicoïdaux par des blocs élastiques. Ces blocs élastiques peuvent être fabriqués à base d'élastomères ou de thermoplastiques adaptés. Ils sont comprimés lorsqu'une pression est exercée sur le bouton poussoir et tendent à retrouver leur forme initiale en repoussant le bouton poussoir dès que cette pression diminue.

Afin de faciliter l'introduction du tube de perfusion, il est conforme à l'invention de prévoir un dispositif de butée limitant la course du bouton poussoir à l'intérieur du boîtier dans la position où les ouvertures latérales du boîtier et le conduit du bouton poussoir sont alignés. Une autre alternative consiste à dimensionner le bouton poussoir de telle manière que, lorsque la face supérieure du bouton poussoir arrive au niveau de l'extrémité de la face supérieure du boîtier, l'alignement des ouvertures des parois du boîtier et du conduit du bouton est atteint. Ces dispositions ont pour fonction de limiter la course du bouton soit par la butée soit par le fait que le doigt exerçant la poussée bute sur le contour du boîtier. L'introduction du tube est ainsi facilitée, car dans cette position maximale, les ouvertures du boîtier et le conduit du bouton sont alignés. De plus, dans cette position extrême, le tube est à nouveau ouvert. Le personnel médical n'a donc pas à chercher la position ouverte en exerçant une pression plus ou moins forte.

Il est conforme à l'invention que la pince ait une forme extérieure de flèche pointant dans la direction de l'axe du tube. Afin de faciliter la détermination du sens de circulation du liquide, la pince peut être installée de sorte que la direction indiquée par la flèche corresponde au sens d'écoulement du liquide dans le tube. Ceci diminue le risque d'erreur de branchement par le personnel médical.

Les dessins annexés permettent d'illustrer l'invention à l'aide d'un exemple.

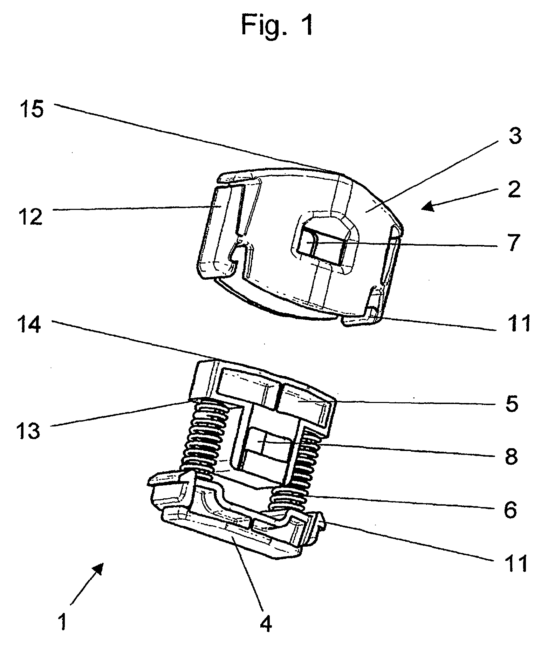

La figure 1 représente un dessin en perspective de la pince avant son montage ;
La figure 2 représente une vue de côté de la pince dans le plan perpendiculaire au tube souple, la pince se trouvant en position ouverte par pression sur le bouton ;
La figure 3 représente une vue en perspective de la pince se trouvant en position ouverte après séparation du socle ;
La figure 4 représente une vue de dessus de la pince, dans le plan perpendiculaire à l'axe du bouton poussoir.

La pince (1) comporte un boîtier (2) constitué de parois latérales (3) et d'un socle (4), un bouton poussoir (5) et un dispositif de ressort (6). Le bouton poussoir (5) est relié au socle (4) par le dispositif de ressort (6). Il est muni d'un conduit transversal (8) qui forme avec les ouvertures (7) des parois latérales (3) du boîtier (2), lorsqu'ils sont alignés, un canal (10) dans lequel peut être introduit un tube souple (9).

Les ouvertures (7) pratiquées dans les parois latérales (3) du boîtier (2) n'ont pas nécessairement toutes les deux la même forme et elles ne sont pas obligatoirement coaxiales. De même, il est envisageable que le contour des ouvertures (7), rectangulaire dans l'exemple présenté ici, soit dans un autre mode de réalisation non fermé par exemple en lui donnant la forme d'un U s'ouvrant du côté du socle (4).

Le socle (4) peut être fixé aux parois (3) du boîtier (2) à l'aide d'un dispositif de verrouillage (11) comportant des pattes extérieures (12) permettant le déverrouillage dudit dispositif (11). Lorsque le socle (4) est fixé au reste du boîtier (2), le dispositif de ressort (6), prenant appui sur le socle (4), exerce sur le bouton poussoir (5) une force tendant à le repousser, ce qui a pour conséquence d'obturer le tube (9) en le pinçant entre les bords du conduit (8) du bouton (5) et les bords des ouvertures (7) des parois (3) du boîtier (2).

On peut agir de deux façons différentes pour supprimer l'obturation du tube.

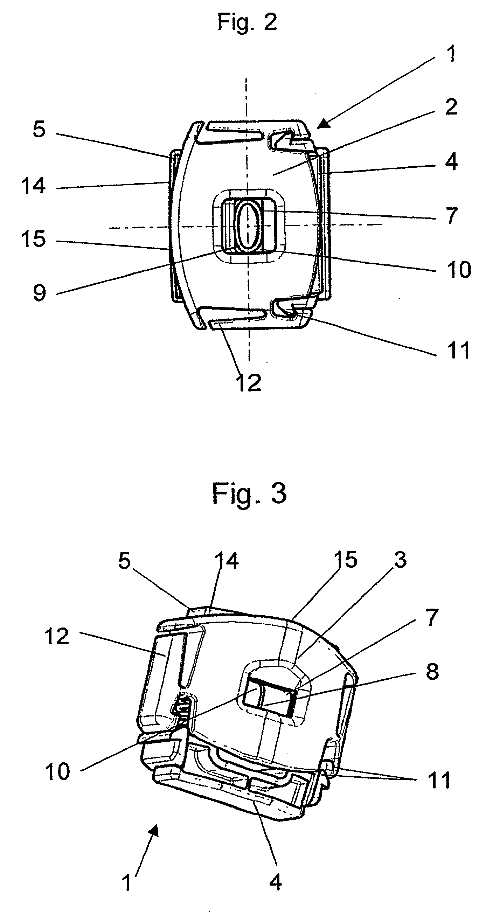

La première consiste, de façon traditionnelle, à exercer sur le bouton poussoir (5) une force opposée à celle exercée par le dispositif de ressort (6). Dans l'exemple envisagé ici, lorsque la face supérieure (14) du bouton poussoir (5) arrive au niveau de l'extrémité de la face supérieure (15) du boîtier (2), les ouvertures (7) des parois (3) du boîtier (2) et le conduit (8) du bouton (5) sont alignés. En appuyant à fond sur le bouton (5), on peut facilement faire pénétrer le tube (9) dans le canal (10) ainsi formé. Selon le même principe, le tube (9) peut s'ouvrir complètement lorsque le bouton poussoir est enfoncé. La figure 2 représente cette situation.

La deuxième façon de supprimer l'obturation consiste à séparer le socle (4) du reste du boîtier (2). Pour cela, il suffit d'exercer une pression sur les pattes extérieures (12) du dispositif de verrouillage (11). Les dimensions du dispositif de ressort (6) sont choisies de telle manière qu'une fois le socle (4) séparé, le canal (10) peut se former librement. C'est la situation montrée à la figure 3.

Le dispositif de ressort peut avoir de nombreuses variantes. L'utilisation de ressorts hélicoïdaux ou de blocs élastiques est la plus courante. Le dispositif peut être constitué d'un seul ressort prenant appui sur le centre du socle (4) et s'appliquant sur le centre du bouton poussoir (5). Pour remplir sa fonction correctement, le ressort a besoin d'une certaine place ce qui oblige à dimensionner la pince de façon conséquente. La solution retenue dans l'exemple citée consiste à utiliser deux ressorts hélicoïdaux placés de part et d'autre du tube (9). Ils prennent appui d'un côté sur le socle (4) et de l'autre sur les ailes intérieures (13) du bouton poussoir (5) qui sont en retrait par rapport au conduit (8). Les dimensions intrinsèques de la pince (1) sont ainsi utilisées au mieux pour placer l'espace nécessaire aux ressorts. Les dimensions générales de la pince (1) peuvent alors être choisies de façon à rendre celle-ci plus compacte.

La forme générale du bouton (5) et du boîtier (2) est choisie de telle sorte que le bouton (5) ne puisse pas pivoter sur son axe afin d'empêcher celui-ci d'être désaxé par rapport aux ouvertures (7). Ceci facilite l'installation du tube (9) dans la pince (1).

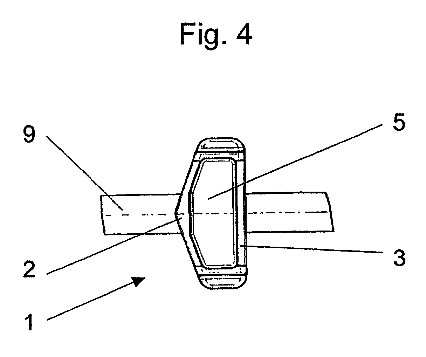

Enfin, vue du dessus la pince (1) présente une forme générale de flèche, comme cela est montré à la figure 4. Grâce à cette disposition, le personnel médical est informé du sens dans lequel circule le liquide.

Le dispositif de ressort (6) étant fixé d'un côté au socle (4) et de l'autre au bouton poussoir (5), aucune pièce ne peut être égarée lorsque la pince (1) est montée sur le tube (9).

A l'exception des ressorts hélicoïdaux, toutes les pièces de la pince y compris les blocs élastiques peuvent être fabriquées en matière plastique. De forme peu compliquée, elles peuvent être facilement produites en grand nombre, de sorte que la pince obtenue est très économique.

La pince selon l'invention présente donc de nombreux avantages par rapport aux pinces du même type actuellement employées :
- La pince peut être maintenue en position ouverte prolongée sans faire appel à une pièce indépendante pouvant être égarée ;
- La suppression de l'obturation peut être obtenue, au moins pour les modes de réalisation simples de l'invention, à l'aide d'une seule main, ce qui peut s'avérer décisif en cas d'urgence ;
- La position ouverte par séparation du socle étant exempte de toute contrainte, la durée de vie de la pince est allongée. Lors du stockage, cette position ouverte ménage aussi bien la pince que le tube qui ne risque pas d'être collé ;
- La forme extérieure de flèche de la pince, en indiquant au personnel médical le sens de circulation du liquide, est un gage supplémentaire de sécurité. L'emboîtement de la pince dans une pompe assure le détrompage lors de l'installation de la pompe ;
- L'ensemble formé par le tube et la pince peut être facilement stérilisé, lorsque le socle est séparé du reste du boîtier.

## Revendications

1. Pince (1) pour obturer un tube souple (9), composée d'un boîtier (2), d'un bouton poussoir pénétrant partiellement à l'intérieur du boîtier et d'un dispositif de ressort, ledit boîtier comprenant un socle (4) et des parois latérales (3) et étant pourvu de deux ouvertures pratiquées dans ses parois latérales, ledit bouton poussoir étant pourvu d'un conduit transversal (8) de sorte que lorsque le conduit du bouton poussoir et les ouvertures latérales (7) du boîtier sont alignés il se forme un canal destiné à recevoir un tube souple, ledit dispositif de ressort, prenant appui sur le socle du boîtier, repoussant le bouton poussoir de façon à pincer le tube souple, **caractérisé en ce que** le socle (4) du boîtier (2) peut être séparé du reste du boîtier (2), permettant ainsi de supprimer de façon réversible la pression exercée sur le bouton poussoir (5) par le dispositif de ressort (6).

2. Pince selon la revendication 1, **caractérisée en ce que** le socle (4) et le boîtier (2) sont munis d'un dispositif de verrouillage (11) pourvu de pattes extérieures (12) permettant de séparer le socle (4) du boîtier (2) lorsqu'une pression est exercée sur lesdites pattes (12).

3. Pince selon la revendication 1 ou 2, **caractérisé en ce que** le socle (4) et le boîtier (2) sont munis d'un dispositif de verrouillage (11) ne pouvant être déverrouillé qu'en exécutant deux opérations consécutives indépendantes qui ne peuvent être accomplies simultanément.

4. Pince selon l'une des revendications précédentes, **caractérisé en ce qu'**elle peut être placée dans la cavité d'une pompe dont le mécanisme assure l'ouverture automatique de la pince (1) uniquement lorsque l'ensemble formé par la pince (1) et le tube (9) est correctement positionné dans ladite pompe.

5. Pince selon l'une des revendications précédentes, **caractérisée en ce que** le boîtier (2) et le bouton poussoir (5) sont munis d'un dispositif empêchant la rotation du bouton poussoir (5) sur son axe de poussé à l'intérieur du boîtier (2).

6. Pince selon l'une des revendications précédentes, **caractérisée en ce que** le dispositif de ressort (6) est fixé par l'une de ses extrémités au socle (4) du boîtier (2) et par son autre extrémité à la face interne (13) du bouton poussoir (5).

7. Pince selon l'une des revendications précédentes, **caractérisée en ce que** le dispositif de ressort (6) est constitué de deux ressorts hélicoïdaux parallèles situés de part et d'autre du conduit transversal (8) du bouton poussoir (5).

8. Pince selon l'une des revendications 1 à 6, **caractérisée en ce que** le dispositif de ressort (6) est constitué de deux blocs élastiques situés de part et d'autre du conduit transversal (8) du bouton poussoir (5).

9. Pince selon l'une des revendications précédentes, **caractérisée en ce qu'**elle est pourvue d'un dispositif de butée limitant la course du bouton poussoir (5) à l'intérieur du boîtier (2) dans la position où les ouvertures latérales (7) du boîtier (2) et le conduit (8) du bouton poussoir (5) sont alignés.

10. Pince selon l'une des revendications 1 à 8, **caractérisée en ce que** les dimensions du bouton poussoir (5) sont choisies de telle manière que, lorsque la face supérieure (14) du bouton poussoir (5) arrive au niveau de l'extrémité de la face supérieure du boîtier (15), les ouvertures latérales (7) du boîtier (2) et le conduit (8) du bouton poussoir (5) sont alignés.

11. Pince selon l'une des revendications précédentes, **caractérisée en ce qu'**elle a une forme extérieure de flèche pointant dans la direction de l'axe du tube (9).

12. Pince selon la revendication 11, **caractérisé en ce que** la direction indiquée par la flèche correspond au sens d'écoulement du liquide dans le tube (9).

## Patentansprüche

1. Klemme (1) zum Abdichten eines weichen Schlauchs (9), bestehend aus einem Gehäuse (2), einem Druckknopf, welcher teilweise ins Innere des Gehäuses dringt, und einer Federvorrichtung, wobei das Gehäuse einen Sockel (4) und seitliche Wände (3) umfasst und mit zwei in den seitlichen Wänden vorgesehenen Öffnungen ausgestattet ist, wobei der Druckknopf mit einer Querführung (8) ausgestattet ist, so dass, wenn die Führung des Druckknopfs und die seitlichen Öffnungen (7) des Gehäuses miteinander ausgerichtet sind, sich ein Kanal bildet, dazu bestimmt, einen weichen Schlauch aufzunehmen, wobei die Federvorrichtung sich gegen den Sockel des Gehäuses anlehnt, den Druckknopf zurückdrückt, um den weichen Schlauch zusammenzudrücken, **dadurch gekennzeichnet, dass** der Sockel (4) des Gehäuses (2) vom Rest des Gehäuses (2) getrennt werden kann, was so ermöglicht, auf reversible Art den durch die Federvorrichtung (6) auf den Druckknopf (5) ausgeübten Druck zu unterdrücken.

2. Klemme gemäß Anspruch 1, **dadurch gekennzeichnet, dass** der Sockel (4) und das Gehäuse (2) mit einer Verriegelungsvorrichtung (11) ausgestattet sind, versehen mit äußeren Beinchen (12), welche das Trennen des Sockels (4) vom Gehäuse (2) ermöglichen, wenn ein Druck auf die Beinchen (12) ausgeübt wird.

3. Klemme gemäß Anspruch 1 oder 2, **dadurch gekennzeichnet, dass** der Sockel (4) und das Gehäuse (2) mit einer Verriegelungsvorrichtung (11) ausgestattet sind, welche nur durch Ausführen zweier unabhängiger aufeinanderfolgender Arbeitsgänge, die nicht gleichzeitig ausgeführt werden können, entriegelt werden kann.

4. Klemme gemäß einem der vorausgegangenen Ansprüche, **dadurch gekennzeichnet, dass** sie in dem Hohlraum einer Pumpe angeordnet werden kann, deren Mechanismus das automatische Öffnen der Klemme (1) sicherstellt einzig dann, wenn die aus der Klemme (1) und dem Schlauch (9) gebildete Gesamtheit korrekt in der Pumpe angeordnet ist.

5. Klemme gemäß einem der vorausgegangenen Ansprüche, **dadurch gekennzeichnet, dass** das Gehäuse (2) und der Druckknopf (5) mit einer Vorrichtung ausgestattet sind, welche die Drehung des Druckknopfs (5) um seine Schubachse im Innern des Gehäuses (2) verhindert.

6. Klemme gemäß einem der vorausgegangenen Ansprüche, **dadurch gekennzeichnet, dass** die Federvorrichtung (6) an einem ihrer Enden am Sockel (4) des Gehäuses (2) befestigt ist und an ihrem anderen Ende an der Innenseite (13) des Druckknopfs (5).

7. Klemme gemäß einem der vorausgegangenen Ansprüche, **dadurch gekennzeichnet, dass** die Federvorrichtung (6) gebildet ist aus zwei parallelen spiralförmigen Federn, angeordnet auf beiden Seiten der Querführung (8) des Druckknopfs (5).

8. Klemme gemäß einem der Ansprüche 1 bis 6, **dadurch gekennzeichnet, dass** die Federvorrichtung (6) gebildet ist aus zwei elastischen Blöcken, angeordnet auf beiden Seiten der Querführung (8) des Druckknopfs (5).

9. Klemme gemäß einem der vorausgegangenen Ansprüche, **dadurch gekennzeichnet, dass** sie mit einer Anschlagsvorrichtung ausgestattet ist, welche den Weg des Druckknopfs (5) im Innern des Gehäuses (2) an der Position begrenzt, wo die seitlichen Öffnungen (7) des Gehäuses (2) und die Führung (8) des Druckknopfs (5) miteinander ausgerichtet sind.

10. Klemme gemäß einem der Ansprüche 1 bis 8, **dadurch gekennzeichnet, dass** die Abmessungen des Druckknopfs (5) so gewählt sind, dass, wenn die obere Seite (14) des Druckknopfs (5) auf Höhe des Endes der oberen Seite des Gehäuses (15) ankommt, die seitlichen Öffnungen (7) des Gehäuses (2) und die Führung (8) des Druckknopfs (5) miteinander ausgerichtet sind.

11. Klemme gemäß einem der vorausgegangenen Ansprüche, **dadurch gekennzeichnet, dass** sie eine äußere Form eines Pfeils hat, welcher in die Richtung der Achse des Schlauchs (9) zeigt.

12. Klemme gemäß Anspruch 11, **dadurch gekennzeichnet, dass** die von dem Pfeil angezeigte Richtung der Ablaufrichtung der Flüssigkeit in dem Schlauch (9) entspricht.

## Claims

1. A clamping device (1) to compress a flexible tube (9), composed of a casing (2), of a push-button reaching partially inside the casing and of a spring-loaded device, said casing (2) including a base (4) and lateral walls (3) and being fitted with two apertures made in its lateral walls, said push-button (5) being provided with a transversal conduit (8) so that when the conduit of the push-button (5) and the lateral apertures of the casing (2) are aligned, a channel is formed, intended to receive a flexible tube, said spring-loaded device (6) resting on the base of the casing, forcing the push-button back in order to clamp the flexible tube, **characterised in that** the base (4) of the casing (2) may be disconnected from the remainder of the casing (2), thereby enabling to release reversibly the pressure exerted onto the push-button (5) by the spring-loaded device (6).

2. A clamping device according to claim 1, **characterised in that** the base (4) and the casing (2) are fitted with a locking device (11) fitted with external lugs (12) enabling to disconnect the base (4) from the casing (2) when a pressure is exerted onto said lugs (12).

3. A clamping device according to claim 1 or 2, **characterised in that** the base (4) and the casing (2) are fitted with a locking device (11) which may only be disconnected by performing two independent consecutive operations which cannot be fulfilled simultaneously.

4. A clamping device according to one of the previous claims, **characterised in that** it may be placed in the cavity of the pump whereof the mechanism enables automatic opening of the clamping device (1) solely when the assembly formed by the clamping device (1) and the tube (9) is positioned correctly in said pump.

5. A clamping device according to one of the previous claims, **characterised in that** the casing (2) and the push-button (5) are fitted with a device which prevents the push-button (5) from rotating on its thrust pin inside the casing (2).

6. A clamping device according to one of the previous claims, **characterised in that** the spring-loaded device (6) is attached by one of its ends to the base (4) of the casing (2) and by its other end to the internal face (13) of the push-button (5).

7. A clamping device according to one of the previous claims, **characterised in that** the spring-loaded device (6) is composed of two parallel helicoid springs situated on either side of the transversal conduit (8) of the push-button (5).

8. A clamping device according to one of the claims 1 to 6, **characterised in that** the spring-loaded device (6) is composed of two elastic blocks situated on either side of the transversal conduit (8) of the push-button (5).

9. A clamping device according to one of the previous claims, **characterised in that** it is fitted with a stopping device, limiting the travel of the push-button (5) inside the casing (2) in the position where the lateral apertures (7) of the casing (2) and the conduit (8) of the push-button (5) are aligned.

10. A clamping device according one of the claims 1 to 8, **characterised in that** the sizes of the push-button (5) are elected so that, when the upper face (14) of the push-button (5) reaches the end of the upper face of the casing (15), the lateral apertures (7) of the casing (2) and the conduit (8) of the push-button (5) are aligned.

11. A clamping device according to one of the previous claims, **characterised in that** it is externally in the form of an arrow pointing in the direction of the axis of the tube (9).

12. A clamping device according to claim 11, **characterised in that** the direction shown by the arrow correspond to the flow direction of the liquid in the tube (9).
